# EUROPEAN PATENT SPECIFICATION

(11) **EP 3 177 755 B1**
(45) Date of publication and mention of the grant of the patent: **27.03.2019**
(21) Application number: 15821540.0
(22) Date of filing: 22.05.2015
(51) Int. Cl.: C25B 3/10, C07C 1/207, C10G 3/00, C07C 6/04, C07C 11/02, C07C 2/00, C07C 6/10

(54) **HIGH PRODUCTIVITY KOLBE REACTION PROCESS FOR TRANSFORMATION OF FATTY ACIDS DERIVED FROM PLANT OIL AND ANIMAL FAT**
HOCHPRODUKTIVER KOLBE-SCHMITT-REAKTIONSPROZESS ZUR TRANSFORMATION VON FETTSÄUREN AUS PFLANZENÖL UND TIERISCHEM FETT
PROCÉDÉ DE RÉACTION DE KOLBE À HAUTE PRODUCTIVITÉ PERMETTANT UNE TRANSFORMATION DES ACIDES GRAS DÉRIVÉS D'UNE HUILE VÉGÉTALE ET DES GRAISSES ANIMALES

(30) Priority: 15.07.2014 US 201414331390
(43) Date of publication of application: 14.06.2017
(73) Proprietor: Advonex International Corp., Brockville, ON K6V 5T4 (CA)
(72) Inventor: JOSHI, Chandrashekhar H., Kingston, Ontario K7K 6K7 (CA); HORNER, Michael Glenn, West Roxbury, Massachusetts 02132-2311 (US); GIBSON, Graham Thomas Thornton, Kingston, Ontario K7K 0C6 (CA); MALEVICH, Dzmitry, Kingston, Ontario K7K 0C1 (CA)
(74) Representative: J A Kemp
(86) International application number: PCT/CA2015/050465
(87) International publication number: WO 2016/008035

(56) References cited:
- US-A1- 2011 024 288
- US-A1- 2011 111 475
- US-A1- 2012 197 050

## Description

### REFERENCE TO RELATED APPLICATIONS

This application is an ***International PCT Application*** that claims priority from non-provisional U.S. Serial No. 14/331,390, filed on July 15, 2014**,** which issued as U.S. Patent No. 8,961,775, on Feb. 24, 2015**.**

### FIELD OF THE INVENTION

Oils from plants and animal fats are hydrolyzed to fatty acids for a Kolbe reaction. The invention relates to a high productivity Kolbe reaction process for electrochemically decarboxylating C4-C28 fatty acids using small amounts of acetic acid to lower anodic passivation voltage and synthesizing C6-C54 hydrocarbons. The C6-C54 undergo olefin metathesis and/or hydroisomerization reaction process to synthesize heavy fuel oil, diesel fuel, kerosene fuel, lubricant base oil, and linear alpha olefin products useful as precursors for polymers, detergents, and other fine chemicals.

### BACKGROUND OF THE INVENTION

The statements in this background section may be useful to an understanding of the invention, but may not constitute prior art.

World-wide production of petroleum is expected to peak around year 2020 and decline thereafter which could cause a global economic decline after 2020. Needed are substitute hydrocarbon sources to petroleum. Inventing alternative, large-scale processes for production of hydrocarbons is needed. These processes need to be economical to be incorporated successfully in free market economies. Research is underway to identify substitute processes and feedstocks for these processes that can be used in large-scale production of needed hydrocarbons. Alternative and renewable feedstocks are being explored for use in economical chemical processes to make hydrocarbons such as fuel oil, diesel fuel, kerosene fuel, lubricant base oil and linear alpha olefins. These particular hydrocarbons are currently obtained from processing petroleum.

Some alternative and renewable feedstocks are plant oils, microbial-produced oils and fatty acids, and animal fats. Because microbial-produced fatty acid feedstocks contain acetates and acetic acid in high molar concentrations of 0.3 M to 0.6 M (Kuhry et al., U.S. Patent No. 8,518,680), the yield of the aforementioned hydrocarbons (fuel oil, diesel fuel, kerosene fuel, lubricant base oil and linear alpha olefins) are very low which indicates that microbial-produced hydrocarbons are not an economically advantageous feedstock. Other prior art teaches the use of a large wt. % of acetic acid (see Weedon et al. (1952); Sumera et al. (1990); and Meresz, U.S. Patent No. 4,018,844, at Col 2:lines 7-10 and in Example 7 in Table I of Col 3-4, which specifically uses acetic acid and oleic acid in a Kolbe electrolysis reaction). Other prior art such as Bradin, U.S. Patent Nos. 7,928,273 and 8,481,771, which teach processes for the production of biodiesel, gasoline and jet fuel start with vegetable oils or animal fat and employ decarboxylation reactions (thermal or Kolbe) of fatty acids, do not mention any use of acetic acid in the decarboxylation reaction.

Feedstocks such as plant oils and animal fats are triglycerides that can be processed using ester hydrolysis, Kolbe electrolysis, olefin metathesis and hydroisomerization to produce fuel oil, diesel fuel, kerosene fuel, lubricant base oil and linear alpha olefins. Ester hydrolysis may be used to convert oils and fats which contain triglycerides to fatty acids. The fatty acids may be decarboxylated and converted into larger hydrocarbons by Kolbe electrolysis. The alkene hydrocarbons produced from Kolbe electrolysis may be reacted by olefin metathesis using catalysts to redistribute the alkenes by a scission and a regeneration of carbon-carbon double bonds. The linear alkene hydrocarbons formed from olefin metathesis using catalysts may be hydroisomerized to add hydrocarbon branches.

There are many plant oils which can be obtained in large amounts from crop plants. Table 1 below indicates the volumetric (liters and gallons) amounts which can be obtained from crops per hectare or acre. Recycled food oils are also being used as a feedstock to produce the aforementioned hydrocarbons.

**Table 1. Amounts of Plant Oils That Have Been Obtained From Various Crop Plants**

| **Crop** | **Liters oil/hectare** | **US gallons/acre** |
|---|---|---|
| corn (maize) | 172 | 18 |
| cashew nut | 176 | 19 |
| oats | 217 | 23 |
| lupine | 232 | 25 |
| kenaf | 273 | 29 |
| calendula | 305 | 33 |
| cotton | 325 | 35 |
| hemp | 363 | 39 |
| soybean | 446 | 48 |
| coffee | 459 | 49 |
| linseed (flax) | 478 | 51 |
| hazelnut | 482 | 51 |
| euphorbia | 524 | 56 |
| pumpkin seed | 534 | 57 |
| coriander | 536 | 57 |
| mustard seed | 572 | 61 |
| camelina | 583 | 62 |
| sesame | 696 | 74 |
| safflower | 779 | 83 |
| rice | 828 | 88 |
| tung oil | 940 | 100 |
| sunflower | 952 | 102 |
| cocoa (cacao) | 1026 | 110 |
| peanut | 1059 | 113 |
| opium poppy | 1163 | 124 |
| rapeseed | 1190 | 127 |
| olive | 1212 | 129 |
| castor bean | 1413 | 151 |
| pecan nut | 1791 | 191 |
| jojoba | 1818 | 194 |
| jatropha | 1892 | 202 |
| macadamia nut | 2246 | 240 |
| brazil nut | 2392 | 255 |
| avocado | 2638 | 282 |
| coconut | 2689 | 287 |
| oil palm | 5950 | 635 |

Plant oils and animal fat (such as beef tallow) contain a mixture of triglycerides which can be hydrolyzed to obtain various fatty acids. Most plant oil-derived and animal fat-derived FFAs typically have 10-20 carbon atoms with zero, one, two or three carbon-carbon double bonds.

The Kolbe electrolysis reaction is a chemical reaction process for the decarboxylation of fatty acids in processes making hydrocarbons. The Kolbe electrolysis reaction process may use a single fatty acid or fatty acid mixtures. A significant renewable source of the fatty acids comes from the hydrolysis of triglycerides of plant oils and animal fats. US2011/024288 describes the Kolbe electrolysis for the synthesis of higher hydrocarbons from fatty acids.

There are problems in using the Kolbe electrolysis reaction to produce hydrocarbons from fatty acids. The problems include a development of a passivation voltage (a voltage drop at the Kolbe cell electrodes during the Kolbe electrolysis reaction) which causes need for a higher cell voltage which results in consumption of large quantities of electricity. If plant oils and animal fats are to be an economically viable source from which hydrocarbons may be produced, then the Kolbe electrolysis reaction needs to be improved in terms of its electrical usage efficiency.

In the prior art, improvements have been attempted in the Kolbe electrolysis reaction process by adding a large wt. % of acetic acid to accompany the fatty acids undergoing decarboxylation (see Weedon et al. (1952); Sumera et al. (1990); and Meresz, U.S. Patent No. 4,018,844, at Col 2:lines 7-10 and in Example 7 in Table I of Col 3-4, which specifically uses acetic acid and oleic acid in a Kolbe electrolysis reaction). In the prior art, others practicing the Kolbe reaction do not add any acetic acid at all. Notable examples are the Bradin, U.S. Patent Nos. 7,928,273 and 8,481,771, which teach a production of biodiesel, gasoline and jet fuel from decarboxylation reactions of fatty acids.

Acetic acid is an expensive reagent. Added acetic acid will react in a Kolbe reaction to produce ethane, which is not liquid at room temperature and hence of less interest for particular applications. Production of ethane in this way consumes large amounts of electricity and increases operating costs. In addition, the added acetic acid will react in a Kolbe reaction with the other free fatty acids present in the reaction, such as the fatty acids obtained by hydrolysis of plant oils or animal fats. This side reaction of acetic acid with other fatty acids is Kolbe reaction hetero-coupling. It has been known that the presence of acetic acid will lower the yield of the hydrocarbons that would be produced by the Kolbe reaction process from the feedstock sources (plant oils and animal fats) used to make the fatty acid. Thus there is an important need to improve the Kolbe electrolysis reaction primary hydrocarbon yield and lower wasteful electrical usage by the Kolbe reaction process.

It is against this background that the various embodiments of the present invention were developed.

### BRIEF SUMMARY OF THE INVENTION

In preferred embodiments, the present invention involves a method of increasing productivity of a Kolbe electrolysis reaction forming a C6 to C54 hydrocarbon or C6 to C54 hydrocarbons ("one or more C6 to C54 hydrocarbons"), the method comprising: combining a C4 - C28 fatty acid or a mixture of C4-C28 fatty acids ("one or more C4 - C28 fatty acids") with a solvent and with an amount of acetic acid to create a reaction mixture, wherein the C4 - C28 fatty acid or the mixture of C4 - C28 fatty acids is between about 80 weight percent about 99.8 weight percent of the total carboxylic acid weight percent in the solvent, and wherein the amount of the acetic acid is between about 0.2 weight percent to about 20 weight percent of the total carboxylic acid weight percent in the solvent; and performing a high productivity Kolbe electrolysis reaction on the reaction mixture to produce the C6 to C54 hydrocarbon or the C6 to C54 hydrocarbons, wherein the acetic acid in the reaction mixture lowers a passivation voltage of an electrode used in the Kolbe electrolysis reaction. The source of the C4 - C28 fatty acid or a mixture of C4 - C28 fatty acids may be a plant oil, an animal fat, or a microbial oil.

In other embodiments of the present invention, the solvent is a C1 to C4 alcohol, methanol, ethanol, propanol, isopropanol, butanol, water, or a mixture thereof, and the solvent is a mixture which contains between about 0.5 percent to about 50 percent water by volume.

In other embodiments of the present invention, the reaction mixture for the Kolbe electrolysis reaction may not be a solution at room temperature.

In other embodiments of the present invention, the C4- C28 fatty acid in the solvent or the mixture of C4- C28 fatty acids in the solvent are reacted with a base to form an amount of a salt of the C4- C28 fatty acid in the solvent or the mixture of C4- C28 fatty acids.

In other embodiments of the present invention, an electrolyte is added to the reaction mixture to improve electrical conductivity of the Kolbe electrolysis reaction. The electrolyte is selected from the group consisting of a perchlorate salt, a *p*-toluenesulfonate salt, a tetrafluoroborate salt, and a mixture thereof.

In other embodiments of the present invention, the Kolbe electrolysis reaction is conducted at a temperature in the range of about 15 °C to about 100 °C.

In other embodiments of the present invention, a pressure other than atmospheric pressure may be imposed on the reaction mixture during the Kolbe electrolysis reaction to change a rate of loss of the solvent, the acetic acid, or a C1-C6 volatile fatty acid.

In other embodiments of the present invention, current supplied to electrodes is 0.05-1.0 amperes/cm2 area of the electrodes.

In other embodiments of the present invention, the reacting surface of the anode electrode is a platinum group metal such as platinum, iridium, palladium, ruthenium, rhodium, or osmium or is a carbon material such as graphite, glassy carbon, baked carbon; or is a mixture of a platinum group metal and a carbon material.

In other embodiments the present invention, the process also involves following the Kolbe electrolysis reaction with an olefin metathesis reaction using a C2-C5 aliphatic alkene or mixtures thereof. The olefin metathesis reaction modifies a chain length of a hydrocarbon and produces a linear alpha olefin or branched hydrocarbons.

In other embodiments the present invention, the process also involves following the Kolbe electrolysis reaction with an ethenolysis reaction using ethene to obtain 1-decene, 1-heptene, 1-butene, 1-octene, 1-hexene and/or 1,4-pentadiene.

In other embodiments the present invention, the process also involves separating the products of the ethenolysis reaction to obtain 1-decene, 1-heptene, 1-butene, 1,4-pentadiene, a diesel fuel, and a heavy fuel oil.

In other embodiments the present invention, the process also involves separating the products from the Kolbe electrolysis reaction which are an amount of diesel fuel and a heavy fuel oil. In other embodiments the present invention also involves hydroisomerizing the heavy fuel oil to produce a lubricant base oil.

In other embodiments of the present invention, the hydroisomerization reaction uses a catalyst which is a silica/alumina-based zeolite containing impregnated platinum, a reaction temperature between about 250 °C to about 400 °C, a reaction pressure between about 10 bar to about 400 bar, and a hydrogen gas to a hydrocarbon ratio of about 2 to about 50.

In other embodiments of the present invention, the reaction mixture uses a solvent and a base from a preceding hydrolysis reaction of a triglyceride.

In other embodiments of the present invention, the concentration of the C4 - C28 fatty acid in the Kolbe electrolysis reaction is between about a 0.01 molar to about a 1 molar.

In preferred embodiments of the present invention, the solvent is methanol, ethanol, or isopropanol. In other preferred embodiments of the present invention, the weight percent of the acetic acid is between 0.2 weight percent to about 5 weight percent.

Other embodiments of the present invention will become apparent from the detailed description of the invention when read in conjunction with the drawings.

### BRIEF DESCRIPTION OF THE DRAWINGS

The invention can be further understood by referring to the drawings which represent certain embodiments of the invention.

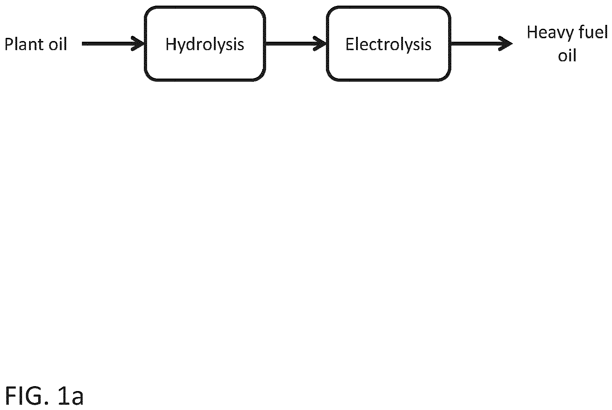

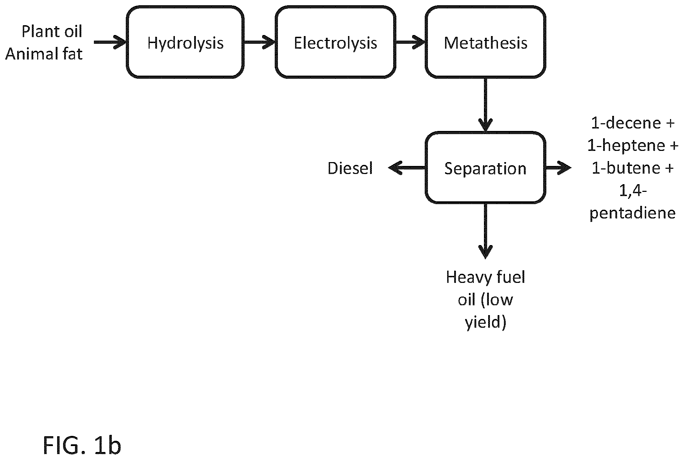

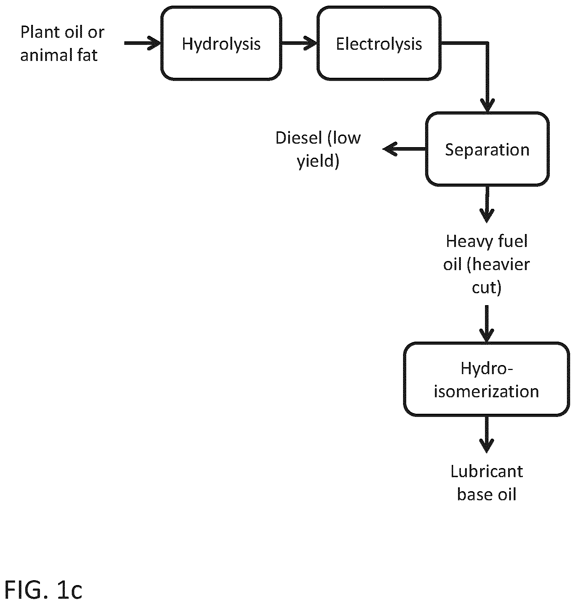

FIGs. 1a, 1b, and 1c depict sequential process steps of the invention for making various hydrocarbons. In FIG. 1a named are sequential hydrolysis and Kolbe electrolysis processes to make hydrocarbons that can be used as a heavy fuel oil. In FIG. 1b named are sequential hydrolysis, Kolbe electrolysis, olefin metathesis, and separation processes to make hydrocarbons suitable for diesel fuel, heavy fuel oil, and shorter linear alpha olefins that can be used as a kerosene fuel or can be used as precursors for advanced polymers, detergents and other fine chemicals. In FIG. 1c named are sequential hydrolysis, Kolbe electrolysis, a separation, and hydroisomerization processes to make hydrocarbons useful as lubricant base oil.
FIG. 2 depicts a hydrolysis reaction of a triglyceride and water to produce free fatty acids and glycerol which is a process step which may be practiced in accordance with certain embodiments of the invention.
FIG. 3 depicts a Kolbe electrolysis reaction of oleic acid, a free fatty acid, with methanol and potassium hydroxide into three different linear hydrocarbons which is a process step which may be practiced in accordance with certain embodiments of the invention.
FIG. 4 depicts an example of the relative contributions of solution resistance, cathodic potential, and anodic potential to the cell voltage occurring during a Kolbe electrolysis reaction of oleic acid in the presence of sodium hydroxide in 70 % ethanol/water by volume.
FIG. 5 depicts a graph plotting the dependence of the cell voltage of a Kolbe electrolysis reaction of oleic acid as a function of the mole percent of acetic acid added.
FIG. 6 depicts a graph plotting the dependence of the cell voltage of a Kolbe electrolysis reaction of stearic acid as a function of the mole percent of acetic acid added.
FIG. 7 depicts a series of three chemical reactions; hydrolysis, Kolbe electrolysis and olefin metathesis using as a feedstock the oil, glyceryl trioleate, to produce a mixture of two linear hydrocarbons in accordance with certain embodiments of the invention.
FIG. 8 depicts a series of three chemical reactions; hydrolysis, Kolbe electrolysis and olefin metathesis using as a feedstock the oil, glyceryl trilinoleate, to produce a mixture of three linear hydrocarbons in accordance with certain embodiments of the invention.

### DETAILED DESCRIPTION OF THE INVENTION

The invention relates to the production of hydrocarbon compositions at least substantially oxygen-free and made from sustainable plant oils, animal fats, microbial oils, and combinations thereof. These hydrocarbon compositions can be used in a wide variety of applications. In particular, the hydrocarbon compositions can be employed as fuel for use in passenger and heavy-duty ground transportation vehicles, such as industrial trucks, railroads and the like, cargo and cruise ships and the like, and in aircraft, such as airplanes, helicopters, and the like. Further, the hydrocarbon compositions can be used as a replacement for heating oil to heat houses and the like.

In one aspect, the invention concerns a specialized use of several chemical processes for making heavy fuel oil, diesel fuel, kerosene fuel, lubricant base oil, and linear alpha olefins useful as precursors, from plant oils, animal fats, or combinations thereof. These chemical processes include hydrolysis, Kolbe electrolysis, olefin metathesis and hydroisomerization. FIG. 1a, FIG. 1b, and FIG 1c depict sequential process steps of the invention for making various hydrocarbons. In FIG. 1a named are sequential hydrolysis and Kolbe electrolysis processes to make hydrocarbons that can be used as a heavy fuel oil. In FIG. 1b named are sequential hydrolysis, Kolbe electrolysis, olefin metathesis, and separation processes to make hydrocarbons suitable for diesel fuel, heavy fuel oil, and shorter linear alpha olefins that can be used as a kerosene fuel or used as precursors for advanced polymers, detergents and other fine chemicals. In FIG. 1c named are sequential hydrolysis, Kolbe electrolysis, separation, and hydroisomerization processes to make hydrocarbons useful as a lubricant base oil.

Two to four of these processes can be combined to make hydrocarbons useful for different applications, including renewable replacements for middle-distillate fuels, heavy fuels, lubricant base oil and linear alpha olefins. Olefin metathesis may be performed prior to hydrolysis, following hydrolysis, or after electrolysis and lead to the same end products. The hydrocarbons produced from these chemical processes are derived from a biological source selected from the group consisting of plant oil, animal fats, microbial oils and combinations thereof and wherein each hydrocarbon composition is at least substantially free of oxygen.

The Kolbe electrolysis reaction is a known useful process for the decarboxylation of fatty acids derived from the hydrolysis of triglycerides of plant oils and animal fats. However, there are problems in using the Kolbe electrolysis reaction to produce hydrocarbons from fatty acids. The problems include a development of a passivation voltage in the Kolbe electrolysis reaction cell which wastes electricity in the Kolbe process.

The hydrolysis can include acid-catalyzed hydrolysis, base-catalyzed hydrolysis or steam hydrolysis and other methods that efficiently convert the plant oil, microbial oil and animal fat into free fatty acids and glycerol. The catalyst used in the hydrolysis reaction can be selected from a wide variety, including acids and bases. Further, the reaction can include the application of heat to improve solubility and accelerate the reaction. The preferred embodiment for large-scale hydrolysis of thermally stable fats and oils is counter-current steam hydrolysis, e.g. the Colgate-Emery process, which efficiently leads to high yields of free fatty acids.

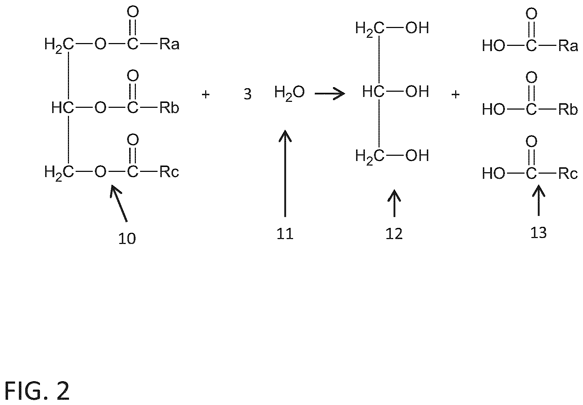

FIG. 2 depicts a chemical structure diagram to show a hydrolysis reaction of a triglyceride and water to produce free fatty acids and glycerol which is a process step which may be practiced in accordance with certain embodiments of the invention. As shown in FIG. 2, a triglyceride 10 is reacted with water 11 to produce glycerol 12 and fatty acids 13. In the reaction example in FIG. 2, the triglyceride 10 includes substituents Rₐ, R_{b}, and Rₑ. A triglyceride is the prevalent component of plant or animal fats. Typical plant oils are a mixture of triglycerides which have commonly three linear hydrocarbon chains as depicted in FIG. 2 by substituents Rₐ, R_{b}, and Rₑ The free fatty acids following hydrolysis of typical plant triglycerides have either 10, 12, 14, 16, 18, 20, 22, or 24 carbons or a mixture of these carbon numbers. The present invention may also use a monoglyceride or a diglyceride. The substituents Rₐ, R_{b}, and R_{c} of the glyceride do not need to have all three substituents be fatty acid esters, e.g., one glyceryl substituent may contain alternative functional groups that include the elements phosphorus, oxygen, or nitrogen.

For some embodiments of the present invention the free fatty acids produced from the hydrolysis reaction contain various even numbers of carbon atoms, from 4 to 28, in a single unbranched chain. Most of the bonds between the carbon atoms in the single fatty acid chain will be single carbon-carbon bonds. When the bonds in the fatty acid chain are all single bonds, then the free fatty acid is called a saturated fatty acid. In unsaturated fatty acids some bonds between adjacent carbon atoms are double bonds. In polyunsaturated fatty acids, the fatty acid chain has multiple double carbon-carbon bonds.

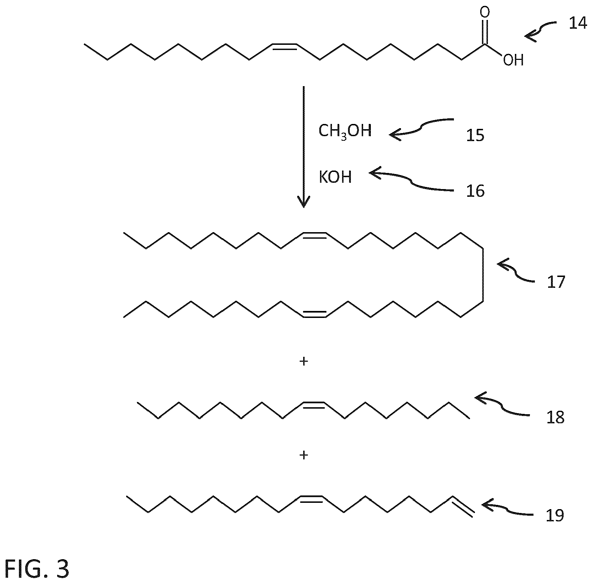

Kolbe electrolysis is a reaction to electrochemically oxidize carboxylic acids to produce alkanes, alkenes, alkane-containing products, alkene-containing products and mixtures thereof. The reaction proceeds through radical intermediates to yield products based on dimerization of these radicals, such that an n-carbon acid will combine with an m-carbon acid to form an alkane and/or alkene of length (m+n-2) carbons along with two carbon dioxide molecules and one hydrogen molecule. The radical intermediates also lead to shorter alkane and/or alkene products by disproportionation. In the Kolbe electrolysis, only the carboxyl groups participate in the reaction and any unsaturation that may be present in the fatty acid chain is preserved in the final product. FIG. 3 shows a Kolbe electrolysis reaction in accordance with certain embodiments of the invention. As shown in FIG. 3, oleic acid 14 is reacted by Kolbe electrolysis in solvent methanol 15 in the presence of base potassium hydroxide 16 to produce (*cis,cis*)-9,25-tetratriacontadiene 17, in addition to small amounts of disproportionation products 18 and 19.

In Kolbe electrolysis, homocoupling is the reaction of two similar free fatty acids creating a symmetrical hydrocarbon product, and heterocoupling is the reaction between two different free fatty acids. The mixture of fatty acids derived from the hydrolysis of a plant oil, microbial oil, animal fat, or combination thereof undergo both homocoupling reactions and heterocoupling reactions in Kolbe electrolysis. The resulting hydrocarbons constitute a range of chain lengths and molecular weights, including those derived from disproportionation of the radical intermediates, which are in the range suitable for diesel fuel, heavy fuel oil and lubricant base oil.

The productivity of the Kolbe reaction is critical to the commercial success of producing hydrocarbons from free fatty acids. The efficiency of the use of electrical current is measured by the current yield, which is the percentage of current used for the reaction of interest (Kolbe electrolysis) relative to the total current applied. For commercial use, however, the cost of electrical power is as important as the current yield, and as such the cell voltage is another critical parameter. The inventors have defined a new term, "productivity", defined herein as the product yield divided by the electrical energy required, in units such as g/kWh. By optimizing this figure, a high productivity is obtained resulting in lower production costs. In the case of Kolbe electrolysis, the specified product for the calculation of productivity is defined as all the hydrocarbons of interest for a specific application derived from the substrate fatty acids.

Products of the hydrolysis reaction which may be present in the Kolbe electrolysis reaction solution may include some unreacted triglycerides, diglycerides, monoglycerides, or glycerol depending upon the feedstock. In the present invention preferably the hydrolysis reaction includes a significant aqueous phase and is designed so that all of the feedstock fats and oils are hydrolyzed into a water-insoluble free fatty acids phase which floats on top of the aqueous phase. Preferably the glycerol byproduct of the hydrolysis reaction fully dissolves into the aqueous phase. In one embodiment of the present invention, the hydrolysis is performed with a base catalyst in a C1-C3 alcohol. It may be advantageous to retain the solvent and/or base from the hydrolysis reaction, for the Kolbe electrolysis reaction.

Preferred solvents for the Kolbe electrolysis include C1-C3 alcohols. More preferably the solvents employed in the Kolbe electrolysis reaction are methanol or ethanol or a mixture of C1-C3 alcohols thereof. The Kolbe reaction is tolerant to the presence of water, and water may be present in this reaction in amounts up to 40% by volume. In certain embodiments, solubility of reaction components may be improved in a solvent system which comprises a mixture of alcohol and water. More preferably the solvent system for the Kolbe reaction comprises about 2% to 50%, about 5% to 45%, about 10% to 40% or about 20% to 30% by volume (water in ethanol).

The initial reaction mixture for the Kolbe electrolysis reaction may not be a solution (with the feedstock and other components dissolved) at room temperature (22 °C). In some embodiments of the present invention, the Kolbe electrolysis may be performed at temperatures below or above room temperature. Preferably the Kolbe electrolysis reaction is conducted at a temperature in the range of about 15 °C to about 100 °C. Higher pressures than atmospheric pressure may be employed to prevent loss of the solvent or a boiling over of the reaction mixture. In instances where a volatile fatty acid is present following the hydrolysis reaction, in some embodiments of the present invention the volatile fatty acid may be allowed to volatilize by lowering the pressure following or during the hydrolysis reaction in order to eliminate volatile fatty acids from being present during the Kolbe electrolysis reaction.

In the Kolbe electrolysis reaction, a base may be added to partially convert the carboxylic acid group of the fatty acids to a carboxylate salt prior to initiating or during the Kolbe reaction undergoing electrolysis. In some embodiments of the present invention preferably the fatty acids will be neutralized by ranges from about 10 to 80, 20 to 60, or 30 to 50 percent. In this case the percent means the concentration of the base in molar units relative to the total carboxylic acid molar concentration. The preferred bases for the neutralization of the fatty acids are hydroxide, alkoxide or carbonate salts of sodium or potassium. Amine bases may also be used. Anions other than the carboxylates of the substrate carboxylic acids may interfere and should not be present. In some embodiments of the present invention an electrolyte may be added to the Kolbe reaction mixture to increase the Kolbe reaction mixture electrical conductivity. Preferably an electrolyte to improve the Kolbe reaction mixture electrical conductivity is selected from the group consisting of perchlorate, *p*-toluenesulfonate or tetrafluoroborate salts of sodium or tetraalkylammonium or a mixture thereof. An increase in mixture conductivity means the same as a decrease in mixture resistivity.

The preferred material of the cathode in Kolbe electrolysis is stainless steel, nickel, or graphite, although other suitable materials may also be used, including platinum or gold. The preferred material of the anode is platinum, at least at the reacting surface of the anode. The anode may be a foil or plate consisting of the preferred anode material or the anode material may be plated on or affixed to a support material such as titanium, graphite, or glass, with the preferred support material being titanium. For example, an anode consisting of a 1 mm-thick titanium plate electroplated with 1 micrometer of platinum was used for the Kolbe electrolysis of oleic acid to give a productivity value equivalent to that found using a platinum foil anode. Other materials may also be used as the anode, including nonporous graphite, gold or palladium.

The preferred current density, defined as the current supplied to the electrode divided by the active surface area of the electrode, applied to the Kolbe electrolysis is 0.05-1.0, 0.1-0.4 or 0.1-0.3 A/cm2.

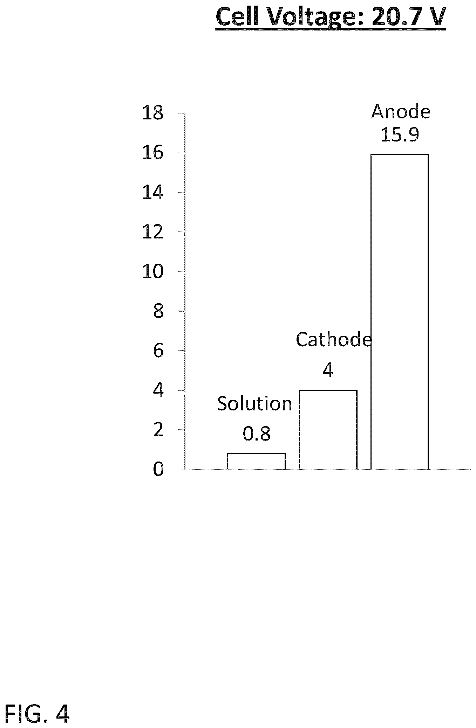

As shown in the Kolbe reaction example depicted in FIG. 4, the net (overall) cell voltage (voltage means electrical potential) of a Kolbe electrolysis reaction cell can be 20.7 volts. The overall cell voltage (also termed the net cell voltage) is a measureable cell voltage between the anode electrode and the cathode electrode. In some embodiments of the present invention as exemplified in Kolbe electrolysis reactions of Examples 1, 2, 3, 4, 5, 6 and 7, the measured cell voltage is between about 12.5 volts to about 33 volts depending upon reaction conditions which include the type of triglyceride, the amount and type of base, the electrical current density, the reaction temperature, and the molar percent acetic acid (less than 10% gives a higher productivity and yield).

This overall cell voltage (cell voltage or net cell voltage) drop measured between the immersed anode and cathode electrodes is believed to be comprised of several voltage drops occurring in the Kolbe electrolysis reaction cells. The cell voltage drop may be due to several factors. These factors in FIG. 4 for example include:
(a) an anode potential drop of 15.9 volts in the example of FIG. 4 which in part is caused by the Kolbe electrolysis reaction with fatty acids and which may change during the Kolbe reaction due to a development of an overvoltage drop on the external immersed electrode surfaces which in part may be caused by an anode electrode passivation process;
(b) a cathode potential drop of 4.0 V in the example of FIG. 4 which may change during the Kolbe reaction due to a development of an overvoltage drop on the external immersed electrode surfaces which in part may be caused by a cathode passivation process;
(c) a Kolbe reaction cell solution voltage drop of 0.8 volts due to solution resistivity and current flow between the electrodes (which according the Ohm's law may be calculated as the mathematical product of the measured solution resistance between the electrodes before the Kolbe reaction solution and the current flow between the electrodes.

The passivation process at the immersed electrodes during the Kolbe reaction is a chemical process problem which needs to be controlled when possible. Passivation may be found to be a voltage-dependent, a current-dependent, or a solution resistivity-dependent factor. The causes and means to modulate electrode passivation may require controlling many Kolbe reaction conditions, and thus controlling passivation may be problematic.

For example, in some embodiments of the present invention, the net cell voltage needs to be minimized to make the Kolbe process economical which means not wasting the electricity going into the Kolbe electrolysis reaction. The net cell voltage is a voltage that can be current-dependent and would be cell resistivity dependent. The electrical current at an adequate electrochemical voltage driving force in the Kolbe reaction cell causes Kolbe electrolysis reaction of free fatty acids to have an adequate yield and reasonable reaction duration. For example, for some embodiments of the present invention, preferably the current flow ranges between about 0.05 to about 0.50 amperes/cm² across the conductive surfaces of the anode and cathode during the Kolbe reaction to have a reaction yield of about 95% and a reaction duration of about one hour. In FIG. 4, the Kolbe cell reaction solution was a 70% ethanol-30% water solution and the fatty acid was 0.5 M oleic acid that has been neutralized by 40 mole percent with sodium hydroxide. The Kolbe electrodes had a 1.5 mm solution gap and the Kolbe reaction was operated with a current density of 0.2 amperes/cm² of electrode area.

In the present invention, passivation at the electrodes employed during the Kolbe electrolysis was considered a wasteful electrical usage problem that needed to be reduced to make commercially practicing the present invention an economically viable process for creating hydrocarbons. However, at the same time for some embodiments of the present invention, the yield of the desired Kolbe hydrocarbon product needed to be a high yield. It was found that the amount of electrode voltage passivation could be reduced by including small amounts of acetic acid or sodium acetate to the Kolbe reaction solution, the acetate or acetic acid being less than 10 mole percent relative to the molar concentration of the fatty acid and which did not greatly reduce the Kolbe reaction yield.

As mentioned by the example in FIG. 4, there are multiple factors which impact the overall cell voltage of a Kolbe electrolysis reaction. The electrochemical potentials for the anodic and cathodic reactions are expected to contribute about 2.5 V and <2 V, respectively, so that the remainder of the voltage measured for the anode and cathode come from electrode passivation. Thus, in the example of FIG. 4, the anodic overvoltage constitutes the majority of the overall cell voltage of 20.7 V. Electrical resistance of the solution can be reduced by adding a supporting electrolyte to the solution, although in example FIG. 4 solution resistance voltage drop is not a significant contributor, accounting for only 0.55 V/millimeter (mm) electrode gap distance. In the example depicted in FIG. 4 there is only a 1.5 millimeter electrode gap.

Electrode passivation has been found to be greater when the fatty acid substrate in the Kolbe reaction is an unsaturated fatty acid with polyunsaturated fatty acids leading to greater passivation than mono-unsaturated fatty acids. A reduction in the effects of passivation, and thus cell voltage, is considered to have an important direct impact on the productivity of the reaction.

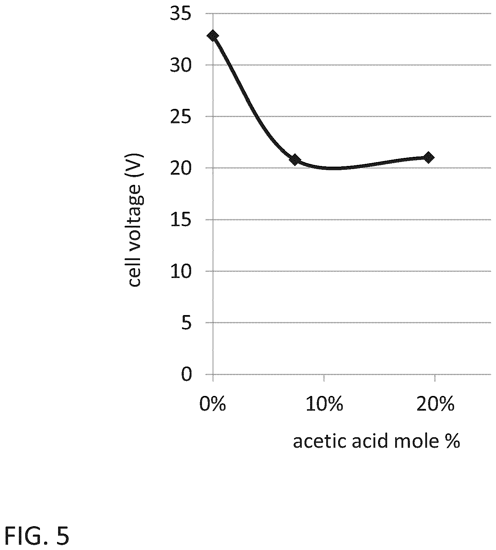

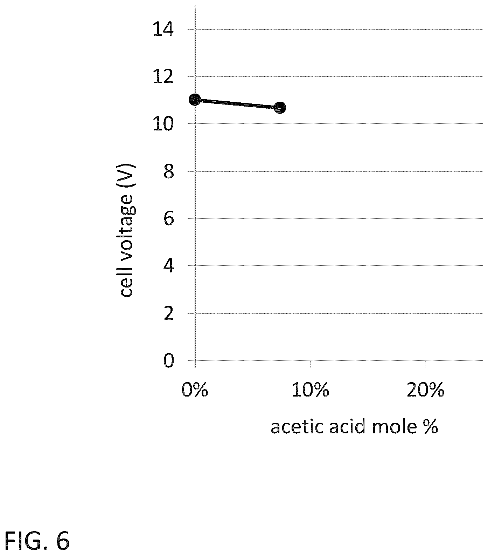

In one aspect of the invention, the productivity of the Kolbe electrolysis reaction is improved by adding a small molar percent acetic acid to the reaction mixture containing a mixture of fatty acids derived from a plant oil, an animal fat, microbial oil or a combination thereof. The addition of acetic acid substantially reduced electrode passivation leading to lower cell voltage and increased productivity. Surprisingly, even small amounts of added acetic acid lead to a considerable reduction in cell voltage, and the dependence of cell voltage on the amount of added acetic acid is non-linear. FIG. 5 shows a graph which plots the dependence of cell voltage on the mole % of acetic acid mixed with oleic acid. From FIG. 5 it can be seen that only 7.4 mole % acetic acid lowers the voltage from 32.8 V in the case of 100% oleic acid to 21.0 V, or to 64% of original. This translates to an increase in productivity (mass per electrical energy used) of 56%. In contrast, for stearic acid, the saturated equivalent of oleic acid, electrode passivation has significantly less impact on the cell voltage. FIG. 6 shows a graph which plots the dependence of cell voltage on the mole % of acetic acid mixed with stearic acid. From FIG. 6 it can be seen that 7.4 mole % acetic acid has very little effect on the cell voltage, lowering it from 11.0 V in the case of 100% stearic acid to 10.7 V, or to 97% of original. Because passivation was not the main contributor to cell voltage, the acetic acid did not make much of a difference, further confirming a) that acetic acid is acting to ameliorate electrode passivation and b) that solution conductivity, as would be increased by an the addition of acetic acid, does not have much effect on cell voltage for this reaction.

The productivity for the Kolbe electrolysis of the mixture, in g/kW·h, can thus be calculated as: P=M·1000/(*i·*V·t), where M is the mass of products of interest generated, in g; i is the electrical current supplied to the electrochemical cell, in amperes; V is the voltage applied to the electrochemical cell, in volts; and t is the time over which current has been supplied, in hours. It follows from the productivity formula that in order to maximize productivity, the incorporation of acetic acid into the product has to be minimized to maximize the M value. Therefore, in the preferred embodiment of this invention the electrochemical conversion of acetic acid is minimized by using a low content of acetic acid in the solution. This use of acetic acid is in contrast to the prior art, which describes the deliberate Kolbe heterocoupling reaction of fatty acids with short-chain acids such as acetic acid (see, for example Meresz et al., U.S. Patent No. 3,932,616), wherein the preferred embodiment is to use a large molar excess of acetic acid in order to maximize the conversion of fatty acids to heterocoupling products with acetic acid.

In accordance with certain embodiments, the invention can include olefin metathesis with ethene (i.e., ethenolysis) or other lower alkene, such as propene, to modify the chain length of the hydrocarbons and to produce linear alpha olefins. In certain embodiments, branched hydrocarbons can be produced, for example, by use of alkenes having two alkyl substituents on one double-bonded carbon, such as 2-methylpropene (isobutylene), in place of ethene in the olefin metathesis reaction. When employed, olefin metathesis can be performed prior to the hydrolysis and Kolbe electrolysis or in-between the hydrolysis and Kolbe electrolysis or, in the preferred embodiment, after Kolbe electrolysis.

Metathesis is a process involving the exchange of a bond (or bonds) between similar interacting chemical species such that the bonding affiliations in the products are closely similar or identical to those in the reactants. Olefin metathesis reactions operate specifically at carbon-carbon double bonds. In such reactions, an olefin described generically as A=A can react with a second olefin, B=B, to yield a cross-over product, A=B. If multiple unsaturated species are available, all possible combinations of cross-over products can typically be obtained, with the product ratio determined largely by the concentrations of the reactants. Internal olefins can be reacted with ethene to produce smaller olefins. This reaction is referred to as ethenolysis, which produces alpha olefins (compounds with terminal double bonds). In certain embodiments, ethenolysis can be performed on the hydrocarbons derived from the Kolbe electrolysis, leading to the linear alpha olefins 1-decene, 1-heptene, 1-butene and 1,4-pentadiene, among others. These linear alpha olefins are useful as precursors to polymers, detergents, and other fine chemicals. In particular, 1-decene, and to a lesser extent 1-heptene and 1-butene, are useful in the production of poly alpha olefins, specifically useful for synthetic lubricants, comprising Group IV of the API classification of lubricant base oils. Alternatively, the shorter-chain hydrocarbons produced in the olefin metathesis reaction may be used as fuel, either left mixed with longer hydrocarbons to improve the cold-flow properties of the mixture of hydrocarbons or separated, e.g. by distillation, and used as a fuel with good cold-flow properties, e.g. as kerosene would be used. In certain embodiments, wherein the olefin metathesis reaction is performed on the plant oil, animal fat or combination thereof, the hydrocarbon product distribution following hydrolysis and Kolbe electrolysis on the mixture would be the same as that wherein the olefin metathesis reaction is performed on the hydrocarbon products of the Kolbe electrolysis reaction.

The olefin metathesis reaction requires a transition metal catalyst. The catalyst may be either heterogeneous or homogeneous with the reaction medium. Common homogeneous catalysts include metal alkylidene complexes as have been described by Schrock, Grubbs, and others. Common heterogeneous metathesis catalysts include rhenium and molybdenum oxides supported on a silica or alumina carrier.

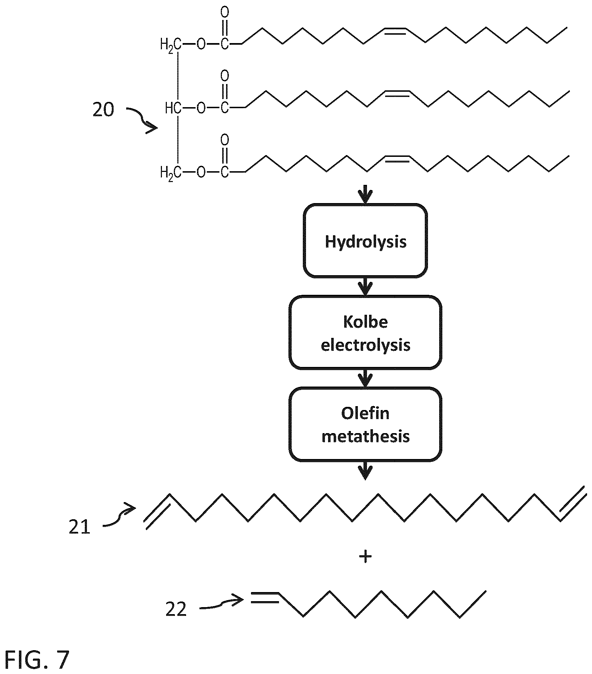

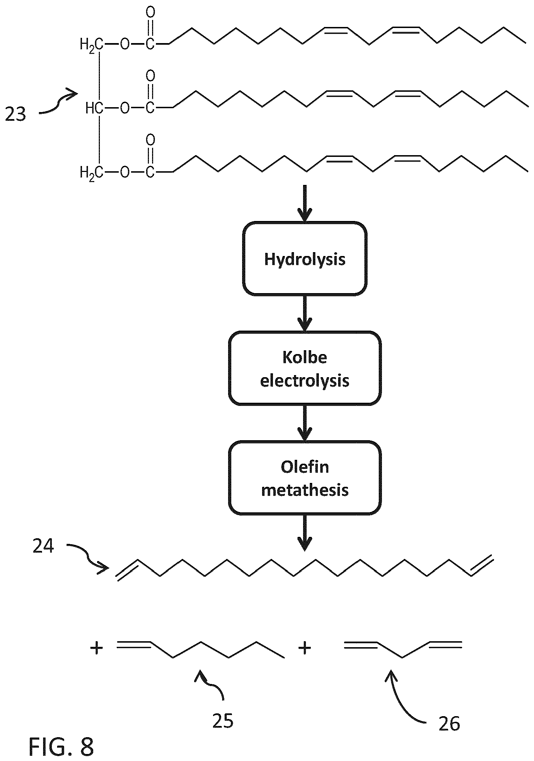

FIG. 7 and FIG. 8 show a process for producing hydrocarbon fuel from hydrocarbons produced by Kolbe electrolysis in accordance with certain embodiments of the invention. As shown in FIG. 7, glyceryl trioleate 20 (a triglyceride) is subjected to hydrolysis, Kolbe electrolysis, and olefin metathesis (ethenolysis) to produce the linear alpha olefins 1,17-octadecadiene 21, and 1-decene 22. As shown in FIG. 8, glyceryl trilinoleate 23 is subjected to hydrolysis, Kolbe electrolysis, and olefin metathesis (ethenolysis) to produce the linear alpha olefins 1,17-octadecadiene 24, 1-heptene 25 and 1,4-pentadiene 26. The composition of linear alpha olefins derived from the olefin metathesis of hydrocarbons derived from oils and fats can be predicted by the average composition of fatty acids in the oil or fat, as the narrow range of fatty acids leads to a narrow range of possible linear alpha olefins. Therefore, the yield of particular desired linear alpha olefins can be improved by careful selection of plant oil, microbial oil, animal fat or combinations thereof wherein the fatty acids that lead to the desired linear alpha olefin are present in high concentration.

In accordance with certain embodiments of the invention, hydroisomerization can be used on the hydrocarbon product of the Kolbe electrolysis reaction to modify the properties of the hydrocarbon such that it is more suitable for use as lubricant base oil. The hydroisomerization reaction is performed in the presence of hydrogen gas and a catalyst having a metal component to catalyze skeletal isomerization, yielding saturated, branched hydrocarbons having about the same molecular weight as the substrate hydrocarbons. The resulting hydrocarbon material is more stable to oxidation and is more fluid at lower temperatures, which are desirable properties. In a preferred hydroisomerization reaction process of the present invention, the catalyst is a silica-alumina zeolite containing impregnated platinum, the temperature is 250-400 °C, the pressure is 10-400 bar, and the H₂:hydrocarbon ratio is 2-50.

### Example 1 - Kolbe electrolysis reaction of beef tallow-derived fatty acids

9.44 parts fatty acids derived from the hydrolysis of beef tallow was added to 89.69 parts methanol; 0.87 parts potassium hydroxide was added to the mixture, which was then heated to 52 °C in a water-jacketed vessel, obtaining a clear solution. An electrolysis cell, consisting of platinum foil anode and nickel cathode separated by 1.5 mm gap, was immersed into the solution. A constant electrical current density of 0.2 A cm⁻² was applied. Within 1 hour, hydrocarbon product separated from the reaction mixture and accumulated at the bottom of the reactor, comprising coupled Kolbe electrolysis products and disproportionation products.

### Example 2 - Kolbe electrolysis reaction of oleic fatty acid in presence of 0.6 wt. % acetic acid of total acids

16.49 parts oleic acid, 0.10 parts acetic acid (0.6 wt. % of total acid), and 0.98 parts sodium hydroxide was dissolved in 59.47 parts ethanol and 22.96 parts water, which was then heated to 50 °C in a water-jacketed vessel. An electrolysis cell, consisting of platinum foil anode and platinum foil cathode separated by 1.5 mm gap, was immersed into the solution. A constant electrical current density of 0.2 A cm⁻² was applied. After 50 minutes of electrolysis an aliquot of the reaction mixture was acidified and extracted into hexane, which was then analyzed by gas chromatography to calculate current yield and productivity (see Table 2).

### Example 3 - Kolbe electrolysis reaction of oleic fatty acid at a moderate electrical current density

16.26 parts oleic acid and 1.15 parts sodium hydroxide was dissolved in 53.52 parts ethanol and 29.07 parts water, which was then heated to 50 °C in a water-jacketed vessel. An electrolysis cell, consisting of platinum foil anode and platinum foil cathode separated by 1.5 mm gap, was immersed into the solution. A constant electrical current density of 0.1 A cm⁻² was applied. After 100 minutes of electrolysis an aliquot of the reaction mixture was acidified and extracted into hexane, which was then analyzed by gas chromatography to calculate current yield and productivity (see Table 2).

### Example 4 - Kolbe electrolysis reaction of oleic fatty acid at a high electrical current density

The solution of Example 3 was electrolyzed in the same manner as in Example 3, except that the current density was 0.3 A cm⁻² and the reaction time was 33.3 minutes.

### Example 5 - Kolbe electrolysis of oleic fatty acid

16.32 parts oleic acid and 0.97 parts sodium hydroxide was dissolved in 53.60 parts ethanol and 29.11 parts water, which was then electrolyzed in the same manner as the solution in Example 2.

### Example 6 - Kolbe electrolysis of more neutralized oleic fatty acid

16.19 parts oleic acid and 2.25 parts sodium hydroxide was dissolved in 52.85 parts ethanol and 28.71 parts water, which was then electrolyzed in the same manner as the solution in Example 2.

### Example 7 - Kolbe electrolysis of oleic fatty acid with a large acetic acid concentration

15.71 parts oleic acid, 6.68 parts acetic acid (29.8 wt. % of total acid), and 3.34 parts sodium hydroxide was dissolved in 48.13 parts ethanol and 26.14 parts water, which was then heated to 40 °C in a water-jacketed vessel. An electrolysis cell, consisting of platinum foil anode and platinum foil cathode separated by 1.5 mm gap, was immersed into the solution. A constant electrical current density of 0.25 A cm⁻² was applied. After 40 minutes of electrolysis an aliquot of the reaction mixture was acidified and extracted into hexane, which was then analyzed by gas chromatography to calculate current yield and productivity (see Table 2).

### Example 8 - Kolbe electrolysis reaction of corn oil-derived fatty acids

10.31 parts fatty acids derived from the hydrolysis of corn oil was added to 88.86 parts methanol; 0.83 parts potassium hydroxide was added to the mixture, which was then heated to 50 °C in a water bath, obtaining a clear solution. An electrolysis cell, consisting of platinum foil anode and nickel cathode separated by 1.5 mm gap, was immersed into the solution. A constant electrical current density of 0.2 A cm⁻² was applied. After 1 hour, the reaction mixture was titrated and the loss of fatty acid was calculated.

### Example 9 - Kolbe electrolysis reaction of corn oil-derived fatty acids in the presence of acetic acid

9.19 parts fatty acids derived from the hydrolysis of corn oil and 0.99 parts acetic acid (9.7 wt. % of total acid) were added to 88.70 parts methanol; 1.11 parts potassium hydroxide was added to the mixture, which was then heated to 50 °C in a water bath, obtaining a clear solution. The reaction was performed as described in Example 8.

As disclosed in the above Examples and in Table 2 data for the Examples, the Kolbe electrolysis reaction of Example 2 which has only 0.7 wt. % of total acid as acetic acid, resulted in the highest productivity value. Thus, Example 2 Kolbe reaction employed a very low molar ratio of acetic acid to oleic acid (a 1:164.9 molar ratio) and the Kolbe reaction current yield of 54.9%. More notably in the presence of trace amounts of less than 1% acetic acid, the Kolbe reaction of Example 2 has a high productivity calculated to be 340 g/kWh. The 50 minute Kolbe reaction of Example 2 produces a high productivity and also a low overall cell voltage of 14.3 volts.

In marked contrast to Example 2, the productivity of Example 7 is inferior despite having 29.8 wt. % of total acid as acetic acid. Example 7 uses 42.7 times more acetic acid than is used in Example 2. In terms of molar ratios, Example 7 uses a molar ratio of acetic acid to oleic acid equal to 15.71:6.68 or a 70%:30% molar percent ratio. The more than 42 fold higher wt. % of acetic acid in Example 7 compared to Example 2 results in a 6.8 fold lower productivity, 50 g/kWh versus 340 g/kWh.

The results of Example 2 with very low molar percent acetic acid in a Kolbe reaction of oleic fatty acid (0.6:99.4 molar percent acetic acid : oleic fatty acid) versus the results of Example 7 with a 70:30 molar percent acetic acid : oleic acid are surprising and contradict the prior art teachings of Kuhry, U.S. Patent No. 8,518,680. For example, Kuhry Example Bio-3 and Table B-1 used 102.4 mM (millimolar) acetic acid to 153.2 total mM acid or a molar percent ratio of 0.66:0.34 molar percent acetic acid:fatty acids (see Col 20:lines 39 to-end of Col. 20). Thus Kuhry teaches using a very high molar fraction. This is also a consequence of Kuhry's use of a source of fatty acids derived from biomass fermentation from which acetic acid is a major acid.

In addition, Bhavaraju et al. (U.S. Patent No. 8,506,789) teaches Kolbe electrolysis reactions containing up to 26 wt. % sodium acetate which is a product of a sodium hydroxide neutralized acid solution, which may be advantageous in providing a high electrolyte conductivity (low Kolbe cell solution resistivity).

As depicted in the Kolbe reaction cell example of FIG. 4 of the present invention, the voltage drop in the Kolbe reaction solution is only 0.8 volts of a 20.7 volt cell (4% of the Kolbe cell voltage example of FIG. 4 of the present invention). Thus for the present invention high acetic acid would insignificantly improve electricity usage (increase productivity g/kWh). Thus the conductivity teaching of Bhavaraju is not relevant to the process of present invention which employs less than 1 wt. % acetic acid. Additionally, Example 7 of the present invention which employed 29.8 wt. % acetic acid gave inferior results which indicate that the Kolbe reaction process of the present invention is markedly different than the Kolbe reaction process of Bhavaraju.

Similarly, the prior art teaches the use of a large wt. % of acetic acid (see Weedon et al. (1952); Sumera et al. (1990); Meresz, U.S. Patent No. 4,018,844 (at Col 2:lines 7-10 and in Example 7 in Table I of Col 3-4, which specifically uses acetic acid and oleic acid in a Kolbe electrolysis reaction). Notably Bradin, U.S. Patent Nos. 7,928,273 and 8,481,771, which teach biodiesel, gasoline and jet fuel production from decarboxylation reactions of fatty acids, however, do not mention using acetic acid at all.

In Example 8, an authentic feedstock of crude fatty acid distillate derived from corn oil leads to a high voltage (46 V) and low productivity, primarily as a result of the large amounts of polyunsaturated fatty acids present in natural corn oil. In the presence of a small amount of acetic acid (9.7 wt. % of total acid), the cell voltage for the Kolbe electrolysis of the same feedstock as Example 8 has dropped to 22.4 V.

**Table 2 - Current yield and productivity data for experiments described in Examples 2-7**

| | Current yield (%) | Cell voltage (V) | Productivity (g/kWh) |
|---|---|---|---|
| Example 2 | 54.9 | 14.3 | 340.0 |
| Example 3 | 66.7 | 24.2 | 244.7 |
| Example 4 | 61.7 | 32.0 | 170.9 |
| Example 5 | 79.5 | 27.9 | 252.5 |
| Example 6 | 29.0 | 12.5 | 206.4 |
| Example 7 | 5.0 | 12.6 | 50.1 |
| Example 8 | 41.8 | 46 | 79.1 |
| Example 9 | 62.7 | 22.4 | 167.6 |

### Example 10 - Ethenolysis of oleic acid-derived Kolbe reaction products in dichloromethane, an olefin metathesis process

3.00 parts hydrocarbons from the Kolbe electrolysis of oleic acid were added to a vessel; 0.62 parts catalyst (commercial Grubbs' 2^{nd} generation metathesis catalyst) was dissolved in 96.38 parts dichloromethane and the resulting solution was added to the vessel. The vessel was sealed and pressurized with ethene to 52 bar. The reaction was stirred at ambient temperature for 2 hours, at which time the vessel was depressurized and ethyl vinyl ether was added to quench the reaction. The contents were passed through silica and analyzed by GC-MS. Results indicate that 1-decene and 1,17-octadecadiene, the only linear alpha olefins expected from a substrate derived entirely from oleic acid, were the only ethenolysis products present in the reaction mixture.

### Example 11 - Hydroisomerization of a straight chain hydrocarbon product of a Kolbe electrolysis reaction to add side-chains to the straight chain hydrocarbon

The product from the Kolbe electrolysis of a fatty acid mixture is passed through a fixed bed reactor containing a catalyst comprising an acidic, porous silica-alumina support (SAPO-11) and 0.5 weight percent platinum loaded onto the support by incipient wetness impregnation. The reactor is heated to 350 °C and pressurized with hydrogen gas to 2 MPa, and the liquid hourly space velocity is 1 h⁻¹.

### Illustrative Embodiments of the Present Invention

Some embodiments of the present invention are a method of increasing productivity of a Kolbe electrolysis reaction forming a C6 to C54 hydrocarbon or C6 to C54 hydrocarbons, the method comprising:
combining a C4 - C28 fatty acid or a mixture of C4- C28 fatty acids with a solvent and with an amount of acetic acid to create a reaction mixture,
wherein the C4 - C28 fatty acid or the mixture of C4- C28 fatty acids is between about 80 weight percent about 99.8 weight percent of the total carboxylic acid weight percent in the solvent, and
wherein the amount of the acetic acid is between about 0.2 weight percent to about 20 weight percent of the total carboxylic acid weight percent in the solvent; and
performing a high productivity Kolbe electrolysis reaction on the reaction mixture to produce the C6 to C54 hydrocarbon or the C6 to C54 hydrocarbons,
wherein the acetic acid in the reaction mixture lowers a passivation voltage of an electrode used in the Kolbe electrolysis reaction. The source of the C4 - C28 fatty acid or a mixture of C4-C28 fatty acids may be a plant oil, an animal fat, or a microbial oil.

Other embodiments of the present invention include wherein the solvent is a C1-to C4 alcohol, methanol, ethanol, propanol, isopropanol, butanol, water, or a mixture thereof, and wherein the solvent is a mixture which contains between about 0.5 percent to about 50 percent water by volume.

Other embodiments of the present invention include wherein the reaction mixture for the Kolbe electrolysis reaction may not be a solution at room temperature.

Other embodiments of the present invention include wherein the C4- C28 fatty acid in the solvent or the mixture of C4- C28 fatty acids in the solvent are reacted with a base to form an amount of a salt of the C4- C28 fatty acid in the solvent or the mixture of C4- C28 fatty acids.

Other embodiments of the present invention include wherein an electrolyte is added to the reaction mixture to improve electrical conductivity of the Kolbe electrolysis reaction, and wherein the electrolyte is selected from the group consisting of a perchlorate salt, a *p*-toluenesulfonate salt, a tetrafluoroborate salt, and a mixture thereof.

Other embodiments of the present invention include wherein the Kolbe electrolysis reaction is conducted at a temperature in the range of about 15 °C to about 100 °C.

Other embodiments of the present invention include wherein a pressure other than atmospheric pressure may be imposed on the reaction mixture during the Kolbe electrolysis reaction to change a rate of loss of the solvent, the acetic acid, or a C1-C6 volatile fatty acid.

Other embodiments of the present invention include wherein current supplied to electrodes is 0.05-1.0 amperes/cm² area of the electrodes

Other embodiments of the present invention include wherein the reacting surface of the anode electrode is a platinum group metal, which includes platinum, iridium, palladium, ruthenium, rhodium, and osmium; or a carbon material, which includes graphite, glassy carbon, baked carbon; or is a mixture of the platinum group metal and the carbon material.

Other embodiments of the present invention further comprise following the Kolbe electrolysis reaction with an olefin metathesis reaction using a C2-C5 aliphatic alkene or mixtures thereof, wherein the olefin metathesis reaction modifies a chain length of a hydrocarbon and produces a linear alpha olefin or branched hydrocarbons.

Other embodiments of the present invention further comprise following the Kolbe electrolysis reaction with an ethenolysis reaction using ethene to obtain 1-decene, 1-heptene, 1-butene, 1-octene, 1-hexene and/or 1,4-pentadiene.

Other embodiments of the present invention further comprise separating the products of the ethenolysis reaction to obtain 1-decene, 1-heptene, 1-butene, 1-octene, 1-hexene, 1,4-pentadiene, a diesel fuel, and a heavy fuel oil.

Other embodiments of the present invention further comprise separating the products from the Kolbe electrolysis reaction which are an amount of diesel fuel and a heavy fuel oil, and further comprising hydroisomerizing the heavy fuel oil to produce a lubricant base oil.

Other embodiments of the present invention include wherein the hydroisomerization reaction uses a catalyst which is a silica/alumina-based zeolite containing impregnated platinum, a reaction temperature between about 250 °C to about 400 °C, a reaction pressure between about 10 bar to about 400 bar, and a hydrogen gas to a hydrocarbon ratio of about 2 to about 50.

Other embodiments of the present invention include wherein the reaction mixture uses a solvent and a base from a preceding hydrolysis reaction of a triglyceride.

Other embodiments of the present invention include wherein the concentration of the C4 - C28 fatty acid in the Kolbe electrolysis reaction is between about 0.01 molar to about 1 molar.

Other embodiments of the present invention include wherein the solvent is methanol. Other embodiments of the present invention include wherein the solvent is ethanol. Other embodiments of the present invention include wherein the solvent is isopropanol. Other embodiments of the present invention include wherein the weight percent of acetic acid is between 0.2 weight percent to about 5 weight percent.

### Unexpected Results Obtained by the Present Invention

One embodiment of the invention is a method of increasing the productivity of a Kolbe electrolysis reaction of fatty acids by using an amount of acetic acid. The productivity is defined as the mass yield of Kolbe electrolysis product relative to the electrical energy used to make it, in units such as grams per kilowatt-hour. This goal is useful for improving the economics of the reaction by reducing the operational costs associated with the manufacture of product.

The Kolbe reaction requires one electron to convert a carboxylic acid into an alkyl radical, which couples with another alkyl radical, the final product thus requiring two electrons. At a given electrical current over a given time, the electrical energy is dependent on the voltage applied to the electrochemical cell. This voltage consists of the anode potential, the cathode potential, and the potential associated with the resistance of the solution between the electrodes. FIG. 4 shows that the potential associated with solution resistance is very small relative to the electrode potential. The electrode potentials are largely comprised of passivation voltage, which arises from the transient coverage of the electrode with material. The role of acetic acid in Applicant's invention is to diminish the effects of electrode passivation.

The inventors obtained an unexpected result, namely, the inventors observed a substantial reduction of Kolbe electrolysis reaction electrode passivation and associated increase in productivity through the addition of small amounts of acetic acid to the Kolbe reaction solution. The added small quantity of acetic acid reduces the Kolbe electrodes' passivation voltage with a negligible effect on the electrolysis product mass yield associated with the incorporation of the acetic acid-derived radicals into the electrolysis product. In contrast to the Inventor's Unexpected Result with low acetic acid in the Kolbe reaction, the use of excess acetic acid in the Kolbe reaction taught by the prior art of Sumera and Bhavaraju had different purposes and results.

One purpose that the prior art teaches for using an excess of acetic acid or acetate salt in the Kolbe reaction is to ensure a rapid and a relatively large production of acetate-derived methyl radicals so that there would be a large degree of coupling by them to fatty acid-derived long-chain alkyl radicals. This purpose and result of using excessive acetic acid in a Kolbe reaction is particularly clearly pointed out by Sumera teaching of composition of Kolbe reaction product Fraction 1 (see Sumera et al., page 338, Table II data, in "Diesel Fuel and Kolbe Electrolysis of Potassium Salts of Coconut Fatty Acids and Acetic Acid," Philippine Journal of Science, Volume 119, No. 4, pages 333-345, 1990, hereinafter "Sumera"). Using gas chromatography data, peaks Nos. 1-5, Sumera discloses in Table II that the result of his excess acetic acid Kolbe reaction, is a product (Fraction 1) which contains 88.88% C10-C18 hydrocarbons. The C10-C18 hydrocarbons are the low-mass products expected when excessive amounts of methyl radicals are made available (as in this case by Sumera) for coupling to fatty acid-derived alkyl radicals. In same chromatography studies of Product (Fraction 1) are peak Nos. 6-8 which Sumera explains, indicate that excess acetate in the Kolbe reaction suppressed high-mass product formation. This is evident since Fraction 1 contains only 11.63% of C20-C24 hydrocarbons, the high-mass products expected if the Kolbe reaction simply caused coupling of the fatty acid-derived alkyl radicals. Bhavaraju also teaches the use of excess acetate salt amounts to promote Kolbe reaction methyl radical formation and methyl radical reactions with fatty acid-derived alkyl radicals (see Bhavaraju et al., U.S. Patent No. 8,506,789, at Col 5:lines 30-46 and Col 14:lines 1-46, hereinafter "Bhavaraju").

A second purpose that the prior art of Bhavaraju teaches is an excess use of acetate salt in the Kolbe reaction solution to create a high electrolyte concentration which will improve the Kolbe reaction solution conductivity between the Kolbe electrodes as much as possible. This teaching means using an amount of acetate salt that is close to the maximum solubility of acetate salt in the reaction solutions. Note Bhavaraju (U.S. Patent No. 8,506,789, Col 5:lines 14-18 and at Col 14:lines 1-6) teaches using sodium acetate up to the maximum solubility of this salt in the reaction solvent (in this case methanol).

Sumera makes statements as to why he prefers to use a large excess of acetic acid. Sumera's statements teach away from the invention. Sumera's Kolbe reaction uses a 2:1 mole ratio of acetate to fatty acid salts. Sumera, at pages 334-5, teaches (quote): "The excess addition of potassium acetate ensured the suppression of solid paraffin production that could form from coupling of fatty acid radicals." At page 336 Sumera adds (quote): "Kolbe electrolysis of these salts of fatty acids [potassium salts of coconut fatty acids] would lead mainly to the production of long chain and high molecular weight alkanes due to coupling of their fatty alkyl radicals." At bottom of page 336 Sumera also teaches (quote): "To avoid long chain fatty alkyl coupling which causes the formation of solid paraffins, acetate ions were introduced to generate methyl radicals could couple with the fatty alkyl radicals. Enough excess of these methyl radicals would suppress the formation of long chain, high molecular weight, solid paraffins." Thus, Sumera teaches a Kolbe electrolysis reaction designed to form as many acetic acid-derived radicals as possible and in as large amounts as possible so as to ensure mainly a coupling of the methyl radicals with the fatty acid-derived long-chain alkyl radicals formed by the reaction, while at the same time preventing the self-coupling of fatty acid-derived long-chain alkyl radicals. Sumera teaches Kolbe reaction conditions ensuring only low-mass hydrocarbon products, whereas quite the opposite, the inventors intend to make only high-mass Kolbe reaction products to achieve high productivity. Accordingly, Sumera conditions teach away from the invention.

The amount of excess acetic acid used by Sumera can be converted to the units of wt. % acetic acid of total carboxylic acid that are used in the present application. Kolbe reaction conditions call for an amount of acetate that is 2 mole equivalents relative to the amount of fatty acid. When calculated, this translates into 36 weight percent of acetic acid in the total carboxylic acid. (36.0 wt. % is calculated from the weight of acetic acid added (0.5535 moles x 60.05 g/mole) divided by the total carboxylic acids weight [(0.5535 moles x 60.05 g/mole)+(0.276 moles x 213.7g/mole)] multiplied by 100%. The molecular weight of acetic acid is 60.05 g/mole. The average molecular weight of coconut oil fatty acids is reported to be 213.7 g/mole (See page 582, second sentence in Results & Discussion section in Petrauskaite et al. (June, 2000), "Physical Refining of Coconut Oil: Effect of crude oil quality and deodorization conditions on neutral oil loss," Journal of the American Oil Chemists Society Vol. 77(6), pp. 581-586, paper no. J9538.)

Experiment data in Example 7 shows that the inclusion of excessive acetic acid (in this case, an amount of 29.8 weight percent of total carboxylic acid) results in a low Kolbe reaction productivity value of 50.1 g/kWh.

On the other hand, the experiment described in Example 2 shows that the inclusion of a very small amount of acetic acid (0.6 weight percent of total carboxylic acid) Unexpectedly Results in a 6.8-fold greater Kolbe reaction productivity of 340.0 g/kWh.

The inventors consider within the calculation of productivity that the product mass yield is an important parameter. The mass of the product is much lower when an excessive amount of acetic acid is used in the Kolbe electrolysis reaction because more of the Kolbe reaction product mass is formed by coupling of low molecular weight acetic acid-derived radicals ("methyl radicals") to (a) other methyl radicals and (b) to fatty acid-derived alkyl radicals. On the other hand, when the Kolbe reaction solution contains only a small amount of acetic acid, then the Kolbe reaction product mass is substantially greater from coupling of high molecular weight fatty acid-derived alkyl radicals to the same. In the Kolbe electrolysis reaction the number of electrons required to generate the methyl radicals from acetic acid (acetate) or to generate the fatty-acid derived alkyl radical is the same and thus not a factor in this analysis.

The unexpected results of Example 2 experiment and very low productivity results of the Example 7 experiment indicate that the invention claimed do not follow the teaching of the Kolbe electrolysis reaction of Sumera. To follow the teaching of Sumera would mean to prefer the methods and the results of the experiment in Example 7 where there is a 6.8-fold lower productivity. Thus Sumera teaches away from goals of the present invention and Sumera's teaching to use 36 wt. % acetic acid by weight of total carboxylic acid goes well beyond the use of 20 wt. %.

As mentioned briefly above, Bhavaraju teaches the use of acetate salts in Kolbe electrolysis reactions (see U.S. Patent No. 8,506,789 at Col 5:lines 14-18 and Col 14:lines 1-6). Bhavaraju suggests both of the uses outlined above, where acetate-derived radicals can be incorporated into the product, or the acetate salt can be used as a supporting electrolyte to increase solution conductivity. Bhavaraju does not indicate a specific useful amount of added acetate salt, but the reasoning for using higher concentrations to target products of mixed coupling of acetate- and fatty acid-derived radicals is the same as outlined above for Sumera. For acetate salt's use as a supporting electrolyte, it is well known to those knowledgeable in the art that higher salt concentration results in higher conductivity. Bhavaraju specifically notes the high solubility limit of sodium acetate in methanol as an advantage (26 percent by weight of solution). Thus, Bhavaraju clearly teaches that higher concentrations of acetate salts are beneficial.

In contrast, as shown by the experimental results described above using Example 2 and Example 7, the use of higher concentrations of acetic acid is not beneficial, as the productivity of the reaction drops considerably (by a factor of 6.8) when 29.8% is used instead of 0.6% acetic acid by weight of total carboxylic acid. Bhavaraju does not include acetate in any of their Examples, nor do their examples indicate a concentration of fatty acids, so there is no specific experiment to which we can compare results. However, the teaching of Bhavaraju suggests maximizing the concentration of sodium acetate (e.g. 26 weight percent sodium acetate in methanol). In comparison, Example 2 uses only 0.14 weight percent (this number is obtained from Example 2 above by taking the weight percent of acetic acid in solution listed there (0.10%) and correcting it by the relative molecular weight of sodium acetate versus acetic acid, i.e. 82.03 g/mole ÷ 60.05 g/mole, as if all added acetic acid were in the form of the sodium salt). In Example 7, which goes well beyond the claimed range of useful acetic acid concentration, the concentration of sodium acetate in solution is 9.1 weight percent (i.e. 6.68 weight percent acetic acid x 82.03/60.05), still well below that taught in Bhavaraju.

Furthermore, from the results in FIG. 4, it can be realized that the solution conductivity is not an important factor in the reduction of cell voltage compared to electrode passivation in our invention, so the advantage of the acetate salt as an electrolyte cited in Bhavaraju is not beneficial in our invention.

There is a major difference between the invention and the teaching of Bhavaraju. If we followed the teaching of Bhavaraju, then adding more acetic acid to react with the fatty acids and/or to increase the solution conductivity in the Kolbe electrolysis reaction will lower the Kolbe reaction productivity. Bhavaraju's teaching to use up to 26% by weight sodium acetate in a Kolbe reaction solution goes well beyond Applicant's claimed range.

The Kolbe reaction methods of Sumera and Bhavaraju respectively teach using high amounts of acetate for (a) promoting Kolbe methyl radical-driven coupling reactions with fatty acid-derived alkyl radicals, (b) suppressing Kolbe fatty acid-derived alkyl radical coupling reactions with fatty acid-derived alkyl radicals, and (c) increasing Kolbe reaction solution conductivity.

One embodiment of the invention, unlike the prior art, performs a Kolbe electrolysis reaction using a low acetic acid wt. % range of 0.2 to 20 wt. % of total carboxylic acid for the purpose of increasing productivity, which is achieved by (a) lowering the Kolbe electrode passivation voltage, (b) lowering Kolbe reaction electrical usage in kilowatt hours (kWh), and (c) promoting Kolbe fatty acid-derived alkyl radical coupling with fatty acid-derived alkyl radicals to produce a high mass product yield. The low wt. % amount of acetic acid does not significantly alter Kolbe reaction solution conductivity, which was shown to have a negligible effect on overall cell voltage in the invention.

Accordingly, it is concluded that the Kolbe reaction methods of Sumera and Bhavaraju teach away from the invention.

### Conclusions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods, constructs and materials are now described.

Where a term is provided in the singular, the inventors also contemplate aspects of the invention described by the plural of that term. As used in this specification and in the appended claims, the singular forms "a", "an" and "the" include plural references unless the context clearly dictates otherwise, e.g., "a tip" includes a plurality of tips. Thus, for example, a reference to "a method" includes one or more methods, and/or steps of the type described herein and/or which will become apparent to those persons skilled in the art upon reading this disclosure.

## Claims

1. A method of increasing productivity of a Kolbe electrolysis reaction forming one or more C6 to C54 hydrocarbons, the method comprising:
combining one or more C4 - C28 fatty acids with a solvent and with an amount of acetic acid to create a reaction mixture,
wherein the one or more C4 - C28 fatty acids is between 80 weight percent 99.8 weight percent of a total carboxylic acid weight percent in the solvent, and
wherein the amount of the acetic acid is between 0.2 weight percent to 20 weight percent of the total carboxylic acid weight percent in the solvent; and
performing a Kolbe electrolysis reaction on the reaction mixture to produce the one or more C6 to C54 hydrocarbons,
wherein the acetic acid in the reaction mixture lowers a passivation voltage of an electrode used in the Kolbe electrolysis reaction.

2. The method of claim 1, wherein the solvent is a C1 to C4 alcohol, methanol, ethanol, propanol, isopropanol, butanol, water, or a mixture thereof, and wherein the solvent contains between 0.5 percent to 50 percent water by volume.

3. The method of claim 1, wherein the reaction mixture for the Kolbe electrolysis reaction is not a solution at room temperature.

4. The method of claim 1, wherein the one or more C4 - C28 fatty acids in the solvent are reacted with a base to form an amount of a salt of the one or more C4 - C28 fatty acids.

5. The method of claim 1, wherein an electrolyte is added to the reaction mixture to improve electrical conductivity of the Kolbe electrolysis reaction, and wherein the electrolyte is selected from the group consisting of a perchlorate salt, a *p*-toluenesulfonate salt, a tetrafluoroborate salt, and mixtures thereof.

6. The method of claim 1, wherein the Kolbe electrolysis reaction is conducted at a temperature in a range of 15 °C to 100 °C.

7. The method of claim 1, wherein a pressure is imposed on the reaction mixture during the Kolbe electrolysis reaction to change a rate of loss of the solvent, or of the acetic acid, or of a C1-C6 volatile fatty acid.

8. The method of claim 1, wherein an electrical current supplied to electrodes is 0.05-1.0 amperes per cm² area of the electrodes.

9. The method of claim 1, wherein the reacting surface of an anode electrode is a platinum group metal, which includes platinum, iridium, palladium, ruthenium, rhodium, and osmium; or a carbon material, which includes graphite, glassy carbon, baked carbon; or mixture of the platinum group metal and the carbon material.

10. The method of claim 1, further comprising:
following the Kolbe electrolysis reaction with an olefin metathesis reaction using a C2-C5 aliphatic alkene or a mixture of C2-C5 aliphatic alkenes, wherein the olefin metathesis reaction modifies a chain length of the hydrocarbon; or
following the Kolbe electrolysis reaction with an ethenolysis reaction using ethene to obtain 1-decene, 1-heptene, 1 -butene, 1-octene, 1-hexene, or 1,4-pentadiene, and, optionally, separating the products of the ethenolysis reaction to obtain 1-decene, 1-heptene, 1-butene, 1-octene, 1-hexene, 1,4-pentadiene, a diesel fuel, and a heavy fuel oil.

11. The method of claim 1, further comprising:
hydroisomerizing at least some of the products from the Kolbe electrolysis reaction to produce a lubricant base oil, optionally, wherein the hydroisomerization reaction uses a catalyst which is a silica/alumina-based zeolite containing impregnated platinum, a reaction temperature between 250 °C to 400 °C, a reaction pressure between 10 bar to 400 bar, and a hydrogen gas to a hydrocarbon ratio of 2 to 50.

12. The method of claim 1, wherein the reaction mixture uses a solvent and a base from a preceding hydrolysis reaction of a triglyceride.

13. The method of claim 1, wherein a concentration of the C4 - C28 fatty acids in the Kolbe electrolysis reaction is between 0.01 molar to 1 molar.

14. The method of claim 1, wherein the solvent is methanol, or ethanol, or a mixture of methanol and ethanol.

15. The method of claim 1, wherein the amount of the acetic acid is between 0.2 weight percent to 5 weight percent.

## Patentansprüche

1. Verfahren des Erhöhens der Produktivität einer Kolbe-Elektrolysereaktion, die einen oder mehrere C6 bis C54 Kohlenwasserstoffe bildet, wobei das Verfahren Folgendes umfasst:
Kombinieren von einer oder mehreren C4-C28 Fettsäuren mit einem Lösungsmittel und mit einer Menge von Essigsäure, um ein Reaktionsgemisch zu erstellen,
wobei die eine oder mehreren C4-C28 Fettsäuren zwischen 80 Gewichtsprozent 99,8 Gewichtsprozent eines Carbonsäure-Gesamtgewichtprozents in dem Lösungsmittel ist und
wobei die Menge der Essigsäure zwischen 0,2 Gewichtsprozent bis 20 Gewichtsprozent des Carbonsäure-Gesamtgewichtprozents in dem Lösungsmittel ist; und
Durchführen einer Kolbe-Elektrolysereaktion auf dem Reaktionsgemisch, um den einen oder die mehreren C6 bis C54 Kohlenwasserstoffe herzustellen,
wobei die Essigsäure in dem Reaktionsgemisch eine Passivierungsspannung einer Elektrode absenkt, die in der Kolbe-Elektrolysereaktion verwendet wird.

2. Verfahren nach Anspruch 1, wobei das Lösungsmittel ein C1 bis C4 Alkohol, Methanol, Ethanol, Propanol, Isopropanol, Butanol, Wasser oder ein Gemisch davon ist und wobei das Lösungsmittel zwischen 0,5 Volumenprozent bis 50 Volumenprozent Wasser enthält.

3. Verfahren nach Anspruch 1, wobei das Reaktionsgemisch für die Kolbe-Elektrolysereaktion bei Raumtemperatur keine Lösung ist.

4. Verfahren nach Anspruch 1, wobei die eine oder mehreren C4-C28 Fettsäuren in dem Lösungsmittel mit einer Base umgesetzt werden, um eine Menge eines Salzes von einer oder mehreren C4-C28 Fettsäuren zu bilden.

5. Verfahren nach Anspruch 1, wobei ein Elektrolyt zu dem Reaktionsgemisch hinzugefügt wird, um die elektrische Leitfähigkeit der Kolbe-Elektrolysereaktion zu verbessern, und wobei der Elektrolyt ausgewählt ist aus der Gruppe bestehend aus einem Perchloratsalz, einem *p*-Toluolsulfonatsalz, einem Tetrafluorboratsalz und Gemischen davon.

6. Verfahren nach Anspruch 1, wobei die Kolbe-Elektrolysereaktion bei einer Temperatur in einem Bereich von 15 °C bis 100 °C ausgeführt wird.

7. Verfahren nach Anspruch 1, wobei ein Druck auf das Reaktionsgemisch während der Kolbe-Elektrolysereaktion auferlegt wird, um eine Verlustrate des Lösungsmittels oder der Essigsäure oder einer C1-C6 flüchtigen Fettsäure zu verändern.

8. Verfahren nach Anspruch 1, wobei ein elektrischer Strom, der Elektroden zugeführt wird, 0,05-1,0 Amper pro cm² Bereich der Elektroden beträgt.

9. Verfahren nach Anspruch 1, wobei die Reaktionsfläche einer Anodenelektrode ein Platingruppenmetall, das Platin, Iridium, Palladium, Ruthenium, Rhodium und Osmium beinhaltet; oder ein Kohlenstoffmaterial, das Graphit, Glaskohlenstoff, gebackenen Kohlenstoff beinhaltet; oder ein Gemisch aus dem Platingruppenmetall und dem Kohlenstoffmaterial ist.

10. Verfahren nach Anspruch 1, ferner umfassend:
Folgen der Kolbe-Elektrolysereaktion mit einer Olefin-Metathese-Reaktion unter Verwendung eines C2-C5 aliphatischen Alkens oder eines Gemischs aus C2-C5 aliphatischen Alkenen, wobei die Olefin-Metathese-Reaktion eine Kettenlänge des Kohlenwasserstoffs modifiziert; oder
Folgen der Kolbe-Elektrolysereaktion mit einer Ethenolysereaktion unter Verwendung von Ethen, um 1-Decen, 1-Hepten, 1-Buten, 1-Octen, 1-Hexen oder 1,4-Pentadien zu erhalten und optional Trennen der Produkte der Ethenolysereaktion, um 1-Decen, 1-Hepten, 1-Buten, 1-Octen, 1-Hexen, 1,4-Pentadien, einen Dieselkraftstoff und ein Schweröl zu erhalten.

11. Verfahren nach Anspruch 1, ferner umfassend:
Hydroisomerisieren von mindestens manchen der Produkte aus der Kolbe-Elektrolysereaktion, um ein Schmierbasisöl herzustellen, optional wobei die Hydroisomerisationsreaktion einen Katalysator, der ein Siliziumdioxid-/Aluminiumoxidbasierter Zeolith ist, der imprägniertes Platin enthält, eine Reaktionstemperatur zwischen 250 °C bis 400 °C, einen Reaktionsdruck zwischen 10 bar bis 400 bar und ein Wasserstoffgas-zu-Kohlenwasserstoff-Verhältnis von 2 bis 50 verwendet.

12. Verfahren nach Anspruch 1, wobei das Reaktionsgemisch ein Lösungsmittel und eine Base von einer vorhergehenden Hydrolysereaktion eines Triglycerids verwendet.

13. Verfahren nach Anspruch 1, wobei eine Konzentration der C4-C28 Fettsäuren in der Kolbe-Elektrolysereaktion zwischen 0,01 Mol bis 1 Mol beträgt.

14. Verfahren nach Anspruch 1, wobei das Lösungsmittel Methanol oder Ethanol oder ein Gemisch aus Methanol und Ethanol ist.

15. Verfahren nach Anspruch 1, wobei die Menge der Essigsäure zwischen 0,2 Gewichtsprozent bis 5 Gewichtsprozent beträgt.

## Revendications

1. Procédé d'augmentation de la productivité d'une réaction d'électrolyse de Kolbe formant au moins un hydrocarbure en C6 à C54, le procédé comprenant :
la combinaison d'au moins un acide gras en C4 à C28 avec un solvant et avec une quantité d'acide acétique pour créer un mélange réactionnel,
l'au moins un acide gras en C4 à C28 étant situé entre 80 pour cent en poids 99,8 pour cent en poids d'un pourcentage total en poids d'acide carboxylique dans le solvant, et
la quantité d'acide acétique étant située entre 0,2 pour cent en poids et 20 pour cent en poids du pourcentage total en poids d'acide carboxylique dans le solvant ; et
la réalisation d'une réaction d'électrolyse de Kolbe sur le mélange réactionnel pour produire l'au moins un hydrocarbure en C6 à C54,
l'acide acétique dans le mélange réactionnel diminuant une tension de passivation d'une électrode utilisée dans la réaction d'électrolyse de Kolbe.

2. Procédé selon la revendication 1, dans lequel le solvant est un alcool en Cl à C4, un méthanol, un éthanol, un propanol, un isopropanol, un butanol, l'eau ou un mélange de ceux-ci, et dans lequel le solvant contient entre 0,5 pour cent et 50 pour cent d'eau en volume.

3. Procédé selon la revendication 1, dans lequel le mélange réactionnel pour la réaction d'électrolyse de Kolbe n'est pas une solution à température ambiante.

4. Procédé selon la revendication 1, dans lequel l'au moins un acide gras en C4 à C28 dans le solvant est mis en réaction avec une base pour former une quantité d'un sel de l'au moins un acide gras en C4 à C28.

5. Procédé selon la revendication 1, dans lequel un électrolyte est ajouté au mélange réactionnel pour améliorer la conductivité électrique de la réaction d'électrolyse de Kolbe, et dans lequel l'électrolyte est choisi dans le groupe constitué par un sel de perchlorate, un sel de *p*-toluènesulfonate, un sel de tétrafluoroborate et des mélanges de ceux-ci.

6. Procédé selon la revendication 1, dans lequel la réaction d'électrolyse de Kolbe est effectuée à une température située dans une plage allant de 15 °C à 100 °C

7. Procédé selon la revendication 1, dans lequel une pression est appliquée sur le mélange réactionnel pendant la réaction d'électrolyse de Kolbe pour modifier un taux de perte du solvant, ou de l'acide acétique ou d'un acide gras volatil en C1 à C6.

8. Procédé selon la revendication 1, dans lequel un courant électrique fourni à des électrodes est de 0,05 à 1,0 ampère par cm² de surface des électrodes.

9. Procédé selon la revendication 1, dans lequel la surface de réaction d'une électrode d'anode est un métal du groupe platine, qui comprend le platine, l'iridium, le palladium, le ruthénium, le rhodium et l'osmium ; ou un matériau carboné, qui comprend le graphite, le carbone vitreux, le carbone cuit ou un mélange de métal du groupe de platine et du matériau carboné.

10. Procédé selon la revendication 1, comprenant en outre :
la poursuite de la réaction d'électrolyse de Kolbe par une réaction de métathèse d'oléfines à l'aide d'un alcène aliphatique en C2 à C5 ou d'un mélange d'alcènes aliphatiques en C2 à C5, la réaction de métathèse d'oléfines modifiant une longueur de chaîne de l'hydrocarbure ; ou
la poursuite de la réaction d'électrolyse de Kolbe par une réaction d'éthénolyse à l'aide d'éthène pour obtenir 1-décène, 1-heptène, 1-butène, 1-octène, 1-hexène, ou 1,4-pentadiène, et, éventuellement, la séparation des produits de la réaction d'éthénolyse pour obtenir 1-décène, 1-heptène, 1-butène, 1-octène, 1-hexène, 1,4-pentadiène, un carburant diesel et une huile de fioul lourd.

11. Procédé selon la revendication 1, comprenant en outre :
l'hydroisomérisation d'au moins une partie des produits provenant de la réaction d'électrolyse de Kolbe pour produire une huile de base lubrifiante, la réaction d'hydroisomérisation utilisant éventuellement un catalyseur qui est une zéolite à base de silice/d'alumine contenant du platine imprégné, une température réactionnelle située entre 250 °C et 400 °C, une pression réactionnelle située entre 10 bars et 400 bars et un gaz d'hydrogène avec un rapport d'hydrocarbure de 2 sur 50.

12. Procédé selon la revendication 1, dans lequel le mélange réactionnel utilise un solvant et une base provenant d'une réaction d'hydrolyse précédente d'un triglycéride.

13. Procédé selon la revendication 1, dans lequel une concentration des acides gras en C4 à C28 dans la réaction d'électrolyse de Kolbe est située entre 0,01 molaire et 1 molaire.

14. Procédé selon la revendication 1, dans lequel le solvant est le méthanol ou l'éthanol ou un mélange de méthanol et d'éthanol.

15. Procédé selon la revendication 1, dans lequel la quantité de l'acide acétique est située entre 0,2 pour cent en poids et 5 pour cent en poids.
